# EUROPEAN PATENT APPLICATION

(11) **EP 4 287 206 A1**
(43) Date of publication of application: **06.12.2023**
(21) Application number: 22796013.5
(22) Date of filing: 14.04.2022
(51) Int. Cl.: G16H 40/40, G16H 40/20, G16H 50/30, G16H 50/70, G06N 20/00

(54) **ELECTRONIC DEVICE AND OPERATION METHOD OF ELECTRONIC DEVICE**

(30) Priority: 29.04.2021 KR 20210055644
(71) Applicant: Samsung Electronics Co., Ltd., Suwon-si, Gyeonggi-do 16677 (KR)
(72) Inventor: PARK, Kwansu, Suwon-si, Gyeonggi-do 16677 (KR)
(74) Representative: Appleyard Lees IP LLP
(86) International application number: PCT/KR2022/005425
(87) International publication number: WO 2022/231179

(57) **Abstract**

An electronic device includes a communication circuit, a sensor, a display, a memory, and a processor. The memory stores one or more instructions that, when executed, may cause the processor to obtain data associated with a health information service by using the sensor, to extract at least one feature from the data based on information stored in the memory and associated with the at least one feature to be used to train a model for determining whether to synchronize the data, to determine whether to synchronize the data based on the model stored in the memory by using the at least one feature, and to send the data to a first external electronic device providing the health information service through the communication circuit, in response to a determination to synchronize the data. Moreover, other embodiments found throughout the present disclosure are also disclosed.

## Description

### [Technical Field]

One or more embodiments of the instant disclosure generally relate to an electronic device and an operating method thereof.

### [Background Art]

With the advancement of mobile electronic devices, increasingly they are able to provide health information services. As such, a health information service provider may be supplied with sensor data collected from the mobile electronic device to provide service to the user. The service provider may process necessary information from the data supplied from the mobile electronic device by using a server and may provide the user with health information service through the mobile electronic device, using the processed information.

### [Disclosure]

### [Technical Problem]

An electronic device may synchronize data by sending collected sensor data to a server that is providing the health information service. In the case where the electronic device performs synchronization frequently, power consumption of the electronic device may increase due to excessive operation of the electronic device, and costs for server management may increase due to the excessive use of the network connecting the electronic device and the server.

However, when transmissions to the server are infrequent to reduce the power consumption and the costs for the server (e.g., when the synchronization period is determined such that synchronization is performed when a specified application is executed or when the synchronization period is determined to be relatively long), the health information service provider may fail to receive the necessary information in a timely fashion from the electronic device collecting the sensor data, thereby causing decrease in the quality of service.

In addition, in the case where the electronic device performs synchronization simply periodically, because the health information service provider selects and uses only the required data of the data synchronized through the server, and does not use the unselected data, the efficiency of resource use of the electronic device and the server may decrease.

### [Technical Solution]

An electronic device according to an embodiment of the disclosure may include a communication circuit, a sensor, a display, a memory, and a processor that is operatively connected with the communication circuit, the sensor, the display, and the memory. The memory stores one or more instructions that, when executed, may cause the processor to obtain data associated with a health information service by using the sensor, to extract at least one feature from the data based on information stored in the memory and associated with the at least one feature to be used to train a model for determining whether to synchronize the data, to determine whether to synchronize the data based on the model stored in the memory by using the at least one feature, and in response to a determination to synchronize the data, to send the data to a first external electronic device providing the health information service through the communication circuit.

Also, an operating method of an electronic device according to an embodiment of the disclosure may include obtaining data associated with a health information service by using a sensor, extracting at least one feature from the data based on information about the at least one feature to be used to train a model for determining whether to synchronize the data, determining whether to synchronize the data based on the model by using the at least one feature, and in response to a determination to synchronize the data, sending the data to a first external electronic device providing the health information service.

### [ Advantageous Effects]

According to various embodiments of the disclosure, the quality of service may be improved by reducing excessive power consumption of an electronic device and costs for server management, when the electronic device uses a health information service, and synchronizing data associated with the health information service in time.

Besides, a variety of effects directly or indirectly understood through this disclosure may be provided .

### [ Description of Drawings]

FIG. 1 is a block diagram of an electronic device in a network environment, according to an embodiment.
FIG. 2 is a block diagram illustrating a program according to an embodiment.
FIG. 3A is a diagram illustrating a synchronization time point of a conventional electronic device.
FIG. 3B is a diagram illustrating a synchronization time point of an electronic device according to an embodiment.
FIG. 4 is a block diagram of an electronic device according to an embodiment.
FIG. 5 is a flowchart illustrating an operation of an electronic device according to an embodiment.
FIG. 6 is a diagram illustrating training data for creating a model to be stored in an electronic device according to an embodiment.
FIG. 7 is a diagram illustrating training data for creating a model to be stored in an electronic device according to an embodiment.
FIG. 8 is a diagram for describing an operation of an electronic device according to an embodiment.
FIG. 9 is a flowchart illustrating an operation of an electronic device according to an embodiment.
FIG. 10 is a diagram for describing an operation of an electronic device according to an embodiment.

With regard to description of drawings, the same or similar components will be marked by the same or similar reference signs.

### [ Mode for Invention]

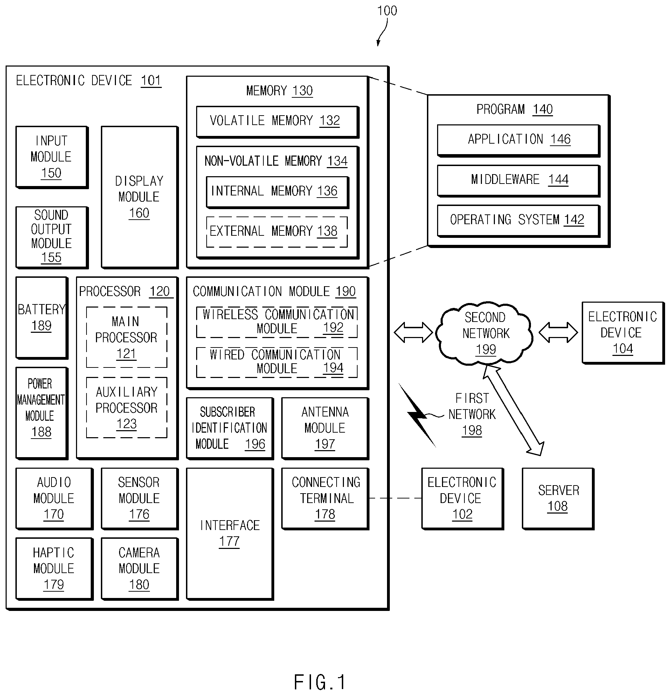

FIG. 1 is a block diagram illustrating an electronic device 101 in a network environment 100 according to an embodiment. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an interperipheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

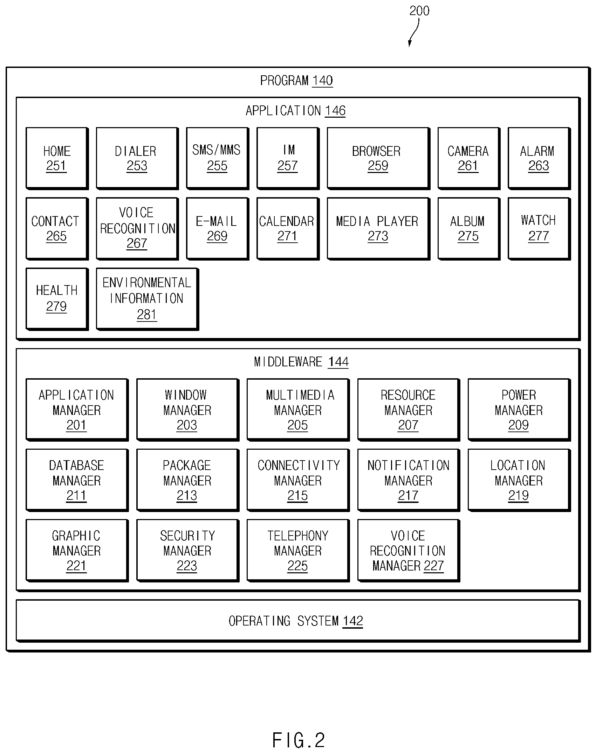

Fig. 2 is a block diagram 200 illustrating the program 140 according to an embodiment. According to an embodiment, the program 140 may include an operating system (OS) 142 to control one or more resources of the electronic device 101, middleware 144, or an application 146 executable in the OS 142. The OS 142 may include, for example, Android^{™}, iOS^{™}, Windows^{™}, Symbian^{™}, Tizen^{™}, or Bada^{™}. At least part of the program 140, for example, may be pre-loaded on the electronic device 101 during manufacture, or may be downloaded from or updated by an external electronic device (e.g., the electronic device 102 or 104, or the server 108) during use by a user.

The OS 142 may control management (e.g., allocating or deallocation) of one or more system resources (e.g., process, memory, or power source) of the electronic device 101. The OS 142, additionally or alternatively, may include one or more driver programs to drive other hardware devices of the electronic device 101, for example, the input module 150, the sound output module 155, the display module 160, the audio module 170, the sensor module 176, the interface 177, the haptic module 179, the camera module 180, the power management module 188, the battery 189, the communication module 190, the subscriber identification module 196, or the antenna module 197.

The middleware 144 may provide various functions to the application 146 such that a function or information provided from one or more resources of the electronic device 101 may be used by the application 146. The middleware 144 may include, for example, an application manager 201, a window manager 203, a multimedia manager 205, a resource manager 207, a power manager 209, a database manager 211, a package manager 213, a connectivity manager 215, a notification manager 217, a location manager 219, a graphic manager 221, a security manager 223, a telephony manager 225, or a voice recognition manager 227.

The application manager 201, for example, may manage the life cycle of the application 146. The window manager 203, for example, may manage one or more graphical user interface (GUI) resources that are used on a screen. The multimedia manager 205, for example, may identify one or more formats to be used to play media files, and may encode or decode a corresponding one of the media files using a codec appropriate for a corresponding format selected from the one or more formats. The resource manager 207, for example, may manage the source code of the application 146 or a memory space of the memory 130.The power manager 209, for example, may manage the capacity, temperature, or power of the battery 189, and determine or provide related information to be used for the operation of the electronic device 101 based at least in part on corresponding information of the capacity, temperature, or power of the battery 189. According to an embodiment, the power manager 209 may interwork with a basic input/output system (BIOS) (not shown) of the electronic device 101.

The database manager 211, for example, may generate, search, or change a database to be used by the application 146. The package manager 213, for example, may manage installation or update of an application that is distributed in the form of a package file. The connectivity manager 215, for example, may manage a wireless connection or a direct connection between the electronic device 101 and the external electronic device. The notification manager 217, for example, may provide a function to notify a user of an occurrence of a specified event (e.g., an incoming call, message, or alert). The location manager 219, for example, may manage locational information on the electronic device 101. The graphic manager 221, for example, may manage one or more graphic effects to be offered to a user or a user interface related to the one or more graphic effects.

The security manager 223, for example, may provide system security or user authentication. The telephony manager 225, for example, may manage a voice call function or a video call function provided by the electronic device 101. The voice recognition manager 227, for example, may transmit a user's voice data to the server 108, and receive, from the server 108, a command corresponding to a function to be executed on the electronic device 101 based at least in part on the voice data, or text data converted based at least in part on the voice data. According to an embodiment, the middleware 244 may dynamically delete some existing components or add new components. According to an embodiment, at least part of the middleware 144 may be included as part of the OS 142 or may be implemented as another software separate from the OS 142.

The application 146 may include, for example, a home 251, dialer 253, short message service (SMS)/multimedia messaging service (MMS) 255, instant message (IM) 257, browser 259, camera 261, alarm 263, contact 265, voice recognition 267, email 269, calendar 271, media player 273, album 275, watch 277, health 279 (e.g., for measuring the degree of workout or biometric information, such as blood sugar), or environmental information 281 (e.g., for measuring air pressure, humidity, or temperature information) application. According to an embodiment, the application 146 may further include an information exchanging application (not shown) that is capable of supporting information exchange between the electronic device 101 and the external electronic device. The information exchange application, for example, may include a notification relay application adapted to transfer designated information (e.g., a call, message, or alert) to the external electronic device or a device management application adapted to manage the external electronic device. The notification relay application may transfer notification information corresponding to an occurrence of a specified event (e.g., receipt of an email) at another application (e.g., the email application 269) of the electronic device 101 to the external electronic device. Additionally or alternatively, the notification relay application may receive notification information from the external electronic device and provide the notification information to a user of the electronic device 101.

The device management application may control the power (e.g., turn-on or turn-off) or the function (e.g., adjustment of brightness, resolution, or focus) of the external electronic device or some component thereof (e.g., a display module or a camera module of the external electronic device). The device management application, additionally or alternatively, may support installation, delete, or update of an application running on the external electronic device.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

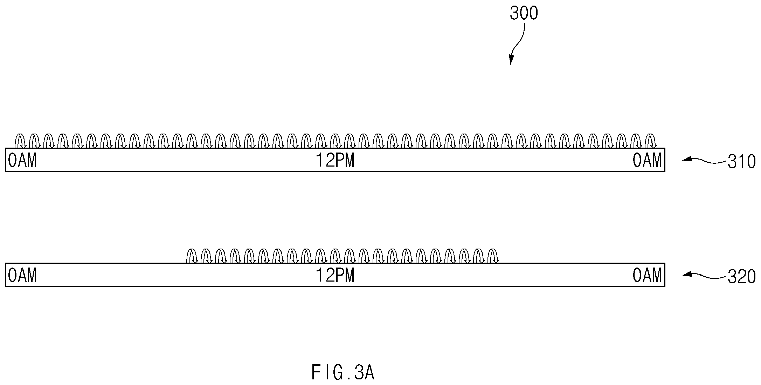

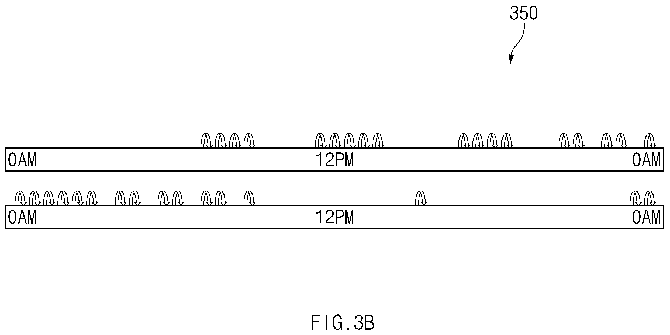

Below, differences between a conventional electronic device and an electronic device according to an embodiment will be described with reference to FIGs. 3A and 3B.

FIG. 3A is a diagram 300 illustrating a synchronization time point of a conventional electronic device. FIG. 3B is a diagram 350 illustrating a synchronization time point of an electronic device according to an embodiment.

The conventional electronic device and the electronic device according to an embodiment may each synchronize data for a health information service, the data obtained by using a sensor, at a specified synchronization time point. For example, each of the conventional electronic device and the electronic device according to an embodiment may process the obtained data and send the processed data to an external electronic device (e.g., a server providing the health information service) at the specified synchronization time point. The specified synchronization time point may be determined by a specified synchronization period.

Referring to first drawing 310 of FIG. 3A, the conventional electronic device may synchronize data based on the specified synchronization period. For example, the conventional electronic device may determine (set or designate) the synchronization period such that data are synchronized regularly at specified time intervals.

Referring to second drawing 320 of FIG. 3A, as another example, the conventional electronic device may determine (set or designate) the synchronization period such that data obtained during a specified time period (or slot) are synchronized.

Although not illustrated in FIG. 3A, as another example, the conventional electronic device may determine the synchronization period such that data are synchronized in response to detecting the occurrence of a specified event. The specified event may be, for example, when data are generated from a sensor or when a specified application is executed.

In the case where the electronic device performs synchronization frequently, power consumption of the electronic device may increase due to excessive operation of the electronic device, and costs for server management may increase due to the excessive use of the network connecting the electronic device and the server.

However, when transmissions to the server are infrequent to reduce the power consumption and the costs for server management (e.g., in the case where the synchronization period is determined such that synchronization is performed when a specified application is executed or in the case where the synchronization period is determined to be relatively long), the health information service provider may fail to receive the necessary information in a timely fashion from the electronic device collecting the sensor data, thereby causing decrease in the quality of service.

In addition, in the case where the electronic device performs synchronization simply periodically, because the health information service provider selects and uses only the required data of the data synchronized through the server, and does not use the unselected data, the efficiency of resource use of the electronic device and the server may decrease.

Referring to FIG. 3B, the electronic device according to an embodiment may perform synchronization by determining (or classifying) whether to synchronize original data, generating data to be sent to the server only with respect to the raw data that has been determined as a synchronization target, and sending the generated data to the server. As the electronic device according to an embodiment obtains data associated with the health information service, the electronic device may determine whether to synchronize obtained data, based on a model created to determine whether to synchronize original data, may generate the to-be-sent data by using only the raw data determined as a synchronization target, and may send the generated data to the server. The electronic device according to an embodiment may improve a personalization (or individuation) characteristic of a model for the user of the electronic device by additionally training an initial model using unique features included in the raw data determined as the synchronization target.

The electronic device according to an embodiment may improve quality of service by reducing excessive power consumption of the electronic device and costs for server management, when providing the health information service, but at the same time synchronize data associated with the health information service in a timely fashion.

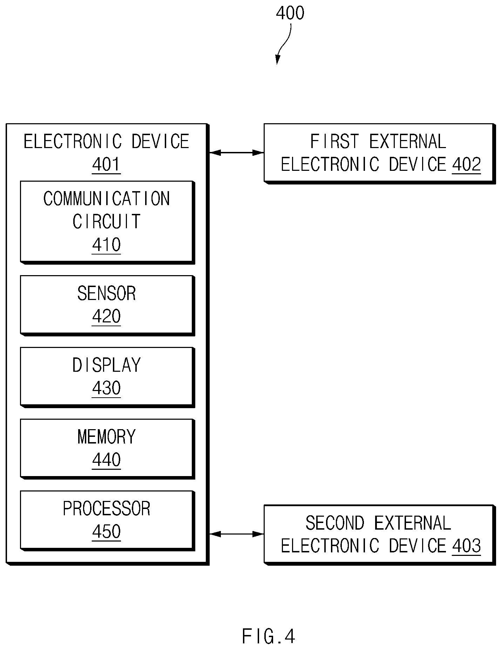

Below, a configuration and operation of the electronic device according to an embodiment will be described with reference to FIG. 4.

FIG. 4 is a block diagram 400 of an electronic device according to an embodiment.

Referring to FIG. 4, an electronic device 401 (e.g., the electronic device 101 of FIG. 1) according to an embodiment may include a communication circuit 410 (e.g., the communication module 190 of FIG. 1), a sensor 420 (e.g., the sensor module 176 of FIG. 1), a display 430 (e.g., the display module 160 of FIG. 1), a memory 440 (e.g., the memory 130 of FIG. 1), and/or a processor 450 (e.g., the processor 120 of FIG. 1). Although not illustrated in FIG. 4, the electronic device 401 may further include at least one of the components of the electronic device 101 illustrated in FIG. 1. For example, the electronic device 401 may further include a camera (not illustrated) (e.g., the camera module 180 of FIG. 1). The camera (not illustrated) may recognize iris information of the user. According to an embodiment, the iris information of the user that the camera recognizes may be used for user authentication. The processor 450 may include a microprocessor or any suitable type of processing circuitry, such as one or more general-purpose processors (e.g., ARM-based processors), a Digital Signal Processor (DSP), a Programmable Logic Device (PLD), an Application-Specific Integrated Circuit (ASIC), a Field-Programmable Gate Array (FPGA), a Graphical Processing Unit (GPU), a video card controller, etc. In addition, it would be recognized that when a general purpose computer accesses code for implementing the processing shown herein, the execution of the code transforms the general purpose computer into a special purpose computer for executing the processing shown herein. Certain of the functions and steps provided in the Figures may be implemented in hardware, software or a combination of both and may be performed in whole or in part within the programmed instructions of a computer. No claim element herein is to be construed under the provisions of 35 U.S.C. 112(f), unless the element is expressly recited using the phrase "means for." In addition, an artisan understands and appreciates that a "processor" or "microprocessor" may be hardware in the claimed disclosure. Under the broadest reasonable interpretation, the appended claims are statutory subject matter in compliance with 35 U.S.C. §101.

According to an embodiment, the communication circuit 410 may establish a communication channel between the electronic device 401 and an external electronic device (e.g., first external electronic device 402 or second external electronic device 403) (e.g., the electronic device 102, the electronic device 104, and the server 108 of FIG. 1) and may perform communication through the established communication channel. According to an embodiment, the first external electronic device 402 may be a server that provides the health information service. According to an embodiment, the second external electronic device 403 may include a wearable electronic device (e.g., smart watch, smart tracker, smart ring, smart glasses, or a smart band) operatively connected with the electronic device 401.

According to an embodiment, the communication circuit 410 may receive data obtained by using a sensor (not illustrated) installed in the second external electronic device 403. For example, the sensor (not illustrated) of the second external electronic device 403 may include a motion sensor and/or a biometric sensor. According to an embodiment, the motion sensor may generate motion data by monitoring the movement, motion, or posture of the user. For example, the motion sensor may include at least one of an acceleration sensor, a gyro sensor, and a geomagnetic sensor. According to an embodiment, the biometric sensor may include at least one of a photoplethysmography (PPG) sensor composed of a light emitting unit and a light receiving unit, or an electrode sensor in contact with the body of the user to exchange electrical signals. According to an embodiment, the biometric sensor may generate various biometric data of the user such as heart rate, skin moisture level, electrocardiogram, body fat percentage, or body composition. According to an embodiment, a processor (not illustrated) of the second external electronic device 403 may convert an analog signal from the sensor (not illustrated) of the second external electronic device 403 to a digital signal, and cause the digital signal to be sent to the electronic device 401 through a communication circuit (not illustrated) of the second external electronic device 403. According to an embodiment, the communication circuit 410 may receive the sensor data (e.g. the aforementioned digital signal) provided from the second external electronic device 403.

According to an embodiment, the communication circuit 410 may send data generated by the electronic device 401 to the first external electronic device 402. According to an embodiment, the data generated by the electronic device 401 may include data that are obtained by processing at least one of the following: data that the processor 450 obtains by using the sensor 420 or data received from the second external electronic device 403 (or data obtained by using the sensor of the second external electronic device 403).

According to an embodiment, the communication circuit 410 may receive, from the first external electronic device 402, a model for determining whether to synchronize the data obtained by using the sensor 420 of the electronic device 401 or the sensor (not illustrated) of the second external electronic device 403 and information about at least one feature to be used to train the model. According to an embodiment, the model may be an equation in which at least one feature is used as an independent variable, a classification value about whether to synchronize is used as a dependent variable, and a quantified correlation (or correlation relationship) between independent variables. According to an embodiment, the model may be generated by the first external electronic device 402, but the disclosure is not limited thereto. For example, the model may be generated by the electronic device 401 or any other external electronic device. According to an embodiment, the first external electronic device 402 may create the model by training the model by using training data in which at least one feature is used as input data and the classification value about whether to synchronize is used as output data.

According to an embodiment, the first external electronic device 402 may collect training data for creating different model for different health information service. According to an embodiment, the kind of data necessary to provide the service, the generation condition (or generating time point) of the data, or the synchronization condition (or synchronization time point) of the data may vary depending on the purpose of the health information service. According to an embodiment, the data may be obtained by using at least one sensor (e.g., acceleration sensor, gyro sensor, geomagnetic sensor, PPG sensor, or electrode sensor). According to an embodiment, the data may include the output value of the at least one sensor. According to an embodiment, the first external electronic device 402 may extract a particular output value of a particular sensor in a plurality of sensors each designated for a different health information services as a feature. According to an embodiment, information indicating whether the output value of a sensor is designated to be extracted as a feature may be referred to as "information about at least one feature to be used to train a model." According to an embodiment, at least one feature may include an output value of at least one sensor designated for a particular health information service. According to an embodiment, the first external electronic device 402 may generate the training data set by extracting an output value of at least one designated sensor, from the data obtained at a plurality of time points. According to an embodiment, the first external electronic device 402 may label a classification value about whether to perform synchronization for the data, on the generated training data set. According to an embodiment, the first external electronic device 402 may train the model by using the training data (or training data set) in which the output value of the at least one sensor designated is used as input data and the labeled classification value about whether to synchronize, which is labeled with respect to input data, is used as output data.

According to an embodiment, the first external electronic device 402 may train the model by using various supervised learning algorithms depending on the complexity of variables of the model. For example, in the case where a correlation relationship between the variables of the model is simple, the first external electronic device 402 may train the model by using a multiple linear regression (MLR) algorithm. As another example, in the case where the correlation relationship of the model is complicated, the first external electronic device 402 may train the model by using a convolutional neural network (CNN) algorithm. As another example, in the case where the feature associated with time flow exists as a variable of the model, the first external electronic device 402 may train the model by using a recurrent neural network (RNN) algorithm.

According to an embodiment, the first external electronic device 402 may train the model by using the multiple linear regression (MLR) algorithm. The model trained by using the MLR algorithm may be, for example, y = b₀ + b_{1·}x₁ + b_{2·}x₂ +...+ b_{n·}xₙ. Here, "y" may be a result; b₀ may be a bias; x₁, x₂... xₙ₋₁, and xₙ may be input variables; b₁, b₂... bₙ₋₁, and bₙ may be weights. According to an embodiment, the first external electronic device 402 may obtain a plurality of equations for the weights b₀ to bₙ by inputting at least one feature to the input variables x₁ to xₙ and inputting a labeled value associated with whether to synchronize to the result "y" and may determine values of the weights b₀ to bₙ by using the plurality of equations. According to an embodiment, the first external electronic device 402 may create the model by using the determined weights b₀ to bₙ. According to an embodiment, the first external electronic device 402 may send the MLR model (y = b₀ + b_{1·}x₁ + b_{2·}x₂ +...+ b_{n·}xₙ) whose weights b₀ to bₙ are determined, to the electronic device 401. According to an embodiment, the communication circuit 410 may receive the model from the first external electronic device 402.

According to an embodiment, the communication circuit 410 may receive the model and information about at least one feature to be used to train the model from the first external electronic device 402. According to an embodiment, the information about the at least one feature may include information for identifying the at least one feature to be used to train the model. According to an embodiment, in the case where a model is "y = b₀ + b_{1·}x₁ + b_{2·}x₂ +...+ b_{n·}xₙ", x₁ to xₙ may correspond to at least one feature, and the information about the at least one feature may be information about whether each of x₁ to xₙ indicates any data or any data component.

According to an embodiment, the sensor 420 may include at least one of a motion sensor, a biometric sensor, a geographic sensor, or a fingerprint sensor. According to an embodiment, the motion sensor may generate motion data by monitoring the motion, movement, or posture of the user. For example, the motion sensor may include at least one of an acceleration sensor, a gyro sensor, and a geomagnetic sensor. According to an embodiment, the biometric sensor may include at least one of a PPG sensor composed of a light emitting unit and a light receiving unit, or an electrode sensor in contact with the body of the user to exchange an electrical signal. According to an embodiment, the biometric sensor may generate various biometric data of the user such as heart rate, skin moisture level, electrocardiogram, body fat percentage, or body composition. According to an embodiment, the geographic sensor may include a device for determining the position of the user, such as a global navigation satellite system (GNSS) receiver, a Wi-Fi module, a Bluetooth module, a near field communication (NFC) module, a visual light communication (VLC) module, an ultra wide-band (UWB) module, or a modem chip detecting a cellular network. According to an embodiment, the geographic sensor may generate data for determining the position of the user. For example, the data for determining the position of the user may include absolute position information such as latitude, longitude, or altitude; context position information such as an address of a specified place, a building name, or a business name; and/or at least one of movement speed or movement direction of the electronic device based on the position information.

According to an embodiment, the fingerprint sensor may recognize fingerprint information of the user. According to an embodiment, the fingerprint information of the user that the fingerprint sensor recognizes may be used for user authentication.

According to an embodiment, the display 430 may display data processed by the processor 450. According to an embodiment, the display 430 may display a user interface. According to an embodiment, the display 430 may include a touch sensor configured to sense touch, or a pressure sensor configured to measure the strength of force generated by the touch. For example, the display 430 may receive a touch input of the user, which is made on the user interface.

According to an embodiment, the memory 440 may store the model and the information about the at least one feature that are received from the first external electronic device 402 through the communication circuit 410. An embodiment in which the model and the information about the at least one feature are received from the first external electronic device 402 and are stored in the memory 440 is described above as an example, but the disclosure is not limited thereto. For example, the model and the information about the at least one feature may be stored in the memory 440 when manufacturing the electronic device 401. According to an embodiment, the memory 440 may store one or more instructions that are executed by the processor 450.

According to an embodiment, the processor 450 may be operatively connected with at least another component of the electronic device 401. According to an embodiment, the processor 450 may be operatively connected with the communication circuit 410, the sensor 420, the display 430, and/or the memory 440. According to an embodiment, the processor 450 may control the operation of the electronic device 401 by executing the one or more instructions stored in the memory 440.

According to an embodiment, the processor 450 may receive a model and information about at least one feature to be used to train the model, from the first external electronic device 402 by using the communication circuit 410. The model and the information about the at least one feature are described above, and thus, duplicative description will be omitted to avoid redundancy. According to an embodiment, the model may be an equation for determining whether to synchronize data obtained by the sensor 420. According to an embodiment, the processor 450 may store the model and the information about the at least one feature received from the first external electronic device 402 in the memory 440.

According to an embodiment, the processor 450 may obtain data associated with the health information service by using the sensor 420. According to an embodiment, the processor 450 may receive the data associated with the health information service from the second external electronic device 403 by using the communication circuit 410. According to an embodiment, the data associated with the health information service may refer to data capable of being used in the health information service.

According to an embodiment, based on the information about the at least one feature stored in the memory 440, the processor 450 may extract the at least one feature from the data obtained by using the sensor 420 or the data received from the second external electronic device 403. According to an embodiment, the processor 450 may extract at least one feature from the data. For example, based on information about at least one feature indicating that the at least one feature includes three axis (e.g., x-axis, y-axis, and z-axis) values of an acceleration sensor, the processor 450 may extract the three axis values of the acceleration sensor from an output value of at least one sensor.

According to an embodiment, the processor 450 may determine whether to synchronize data, based on the model stored in the memory 440 by using the extracted at least one feature. According to an embodiment, the model stored in the memory 440 may be the MLR model. For example, the model stored in the memory 440 may be "y = b₀ + b_{1·}x₁ + b_{2·}x₂ +...+ b_{n·}xₙ". According to an embodiment, the processor 450 may input the extracted at least one feature to the input variables x₁ to xₙ corresponding to each feature. For example, based on the information about the at least one feature indicating that x₁ is an x-axis value of the acceleration sensor, x₂ is a y-axis value of the acceleration sensor, and x₃ is a z-axis value of the acceleration sensor, the processor 450 may input the x-axis value of the acceleration sensor extracted from the data to x₁, may input the y-axis value of the acceleration sensor extracted from the data to x₂, and may input the z-axis value of the acceleration sensor extracted from the data to x₃. According to an embodiment, the processor 450 may determine whether to synchronize data, depending on whether the result "y" calculated by inputting the at least one feature extracted from the data to the input variables x₁ to xₙ is greater than or equal to a specified value (e.g., y₀) or is smaller than the specified value. For example, when the result "y" is greater than or equal to the specified value, the processor 450 may determine to synchronize data; when the result "y" is smaller than the specified value, the processor 450 may determine not to synchronize data.

According to an embodiment, the processor 450 may calculate a reliability of the value indicating whether to synchronize data, which is determined based on the model. According to an embodiment, the processor 450 may determine the reliability of the result of predicting whether to synchronize, based on a difference between the result "y" calculated by inputting the at least one feature extracted from the data to the input variables x₁ to xₙ and the specified value for determining whether to synchronize. According to an embodiment, as the difference between the calculated result "y" and the specified value becomes greater, the reliability may increase.

According to an embodiment, the processor 450 may determine whether the reliability of the value indicating whether to synchronize, which is determined based on the model, is greater than or equal to a specified threshold value or is smaller than the specified threshold value. According to an embodiment, based on whether the difference between the calculated result "y" and the specified value is greater than or equal to the specified threshold value or is smaller than the specified threshold value, the processor 450 may determine whether the reliability of the value indicating whether to synchronize, which is determined based on the model, is greater than or equal to the specified threshold value or is smaller than the specified threshold value. According to an embodiment, based on that the difference between the calculated result "y" and the specified value is greater than or equal to the specified threshold value, the processor 450 may determine that the reliability is greater than or equal to the specified threshold value; based on that the difference between the calculated result "y" and the specified value is smaller than the specified threshold value, the processor 450 may determine that the reliability is smaller than the specified threshold value.

According to an embodiment, when the reliability is smaller than the specified threshold value, the processor 450 may postpone the synchronization of data. According to an embodiment, when the reliability is smaller than the specified threshold value, the processor 450 may display a user interface requesting feedback of the user whether to synchronize data, on the display 430. According to an embodiment, the processor 450 may receive an input indicating (or directing) to synchronize data, from the user through the user interface. According to an embodiment, as the processor 450 receives the input indicating (or directing) data synchronization through the user interface, the processor 450 may synchronize data. According to an embodiment, as the processor 450 receives an input indicating no data synchronization through the user interface, even though data synchronization is determined as being performed based on the model, the processor 450 may not synchronize data.

According to an embodiment, the processor 450 may synchronize the data determined as a synchronization target. According to an embodiment, after processing the data determined as the synchronization target, the processor 450 may send the processed data to the first external electronic device 402.

According to an embodiment, the processor 450 may receive contents of the health information service from the first external electronic device 402. According to an embodiment, the first external electronic device 402 may generate the contents of the health information service based on data about the health information service provided from the electronic device 401 and may send the contents of the health information service to the electronic device 401. The contents of the health information service may include, for example, the result of analyzing data provided from the electronic device 401, or recommended contents based on the data (e.g., workout video, workout plan, expert health advice, or goal setting).

According to an embodiment, the processor 450 may generate training data, in which at least one feature extracted from data whose synchronization is determined based on a model or a user input is used as input data and a value indicating whether to synchronize thus determined is used as output data. According to an embodiment, the processor 450 may train the model by using the generated training data.

According to an embodiment, in the case where whether to synchronize data is determined based on a model, the processor 450 may generate training data in which at least one feature extracted from the data is used as input data and a value indicating whether to synchronize thus determined is used as output data.

According to another embodiment, in the case where the reliability of the value indicating whether to synchronize data, which is determined based on a model, is smaller than the specified threshold value, the processor 450 may display a user interface requesting feedback of the user and may generate training data in which at least one feature extracted from the data is used as input data and the value indicating whether to synchronize determined based on an input received through the user interface is used as output data.

According to another embodiment, the processor 450 may receive an input allowing data to be synchronized from the user, regardless of the determination to synchronize based on the model. For example, the processor 450 may receive an input indicating (or directing) synchronization of data of a time point designated by the user (i.e., data obtained by using a sensor at the designated time point). According to an embodiment, as the processor 450 receives an input allowing data to be synchronized from the user, the processor 450 may extract at least one feature from the data based on information about the at least one feature stored in the memory 440 and may generate training data in which the extracted at least one feature is used as input data and the value indicating that the data is targeted for synchronization is used as output data.

According to an embodiment, the processor 450 may train the model by using the generated training data. According to an embodiment, the processor 450 may additionally train the initial model stored in the memory 440 by using the generated training data. For example, the initial model stored in the memory 440 may be "y = b₀ + b_{1·}x₁ + b_{2·}x₂ +... + b_{n·}xₙ", the bias of the initial model may be b₀, and weights of the initial model may be b₁ to bₙ. According to an embodiment, the processor 450 may obtain a new bias b₀' and new weights b₁' to bₙ' by additionally training the model. According to an embodiment, the processor 450 may obtain an updated model "y = b₀' + b₁'·x₁ + b₂'·x₂+...+ bₙ' xₙ" by additionally training the model by using training data. According to an embodiment, the bias b₀' and the weights b₁' to bₙ' that are obtained as the processor 450 trains the model may be bias and weights to which a unique characteristic of the user is applied. According to an embodiment, the processor 450 may personalize the model by additionally training the model based on data to which the unique characteristic of the user of the electronic device 401 is applied.

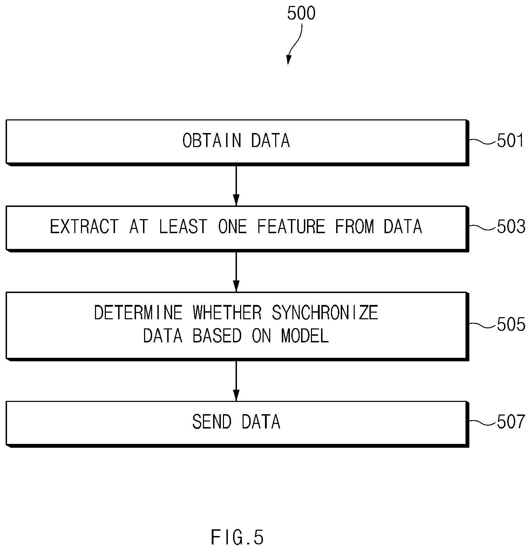

Below, an operation of an electronic device according to an embodiment will be described with reference to FIG. 5.

FIG. 5 is a flowchart 500 illustrating an operation of an electronic device according to an embodiment. Operations of an electronic device to be described below may be performed by the electronic device 401 of FIG. 4 or the processor 450 of the electronic device 401.

In operation 501, the electronic device may obtain data. According to an embodiment, the processor 450 may obtain data associated with the health information service by using a sensor (e.g., the sensor 420 of FIG. 4). According to an embodiment, the electronic device may receive data obtained by using a sensor of a second external electronic device (e.g., the second external electronic device 403 of FIG. 4) from the second external electronic device through a communication circuit (e.g., the communication circuit 410 of FIG. 4).

In operation 503, the electronic device may extract at least one feature from the data. According to an embodiment, the electronic device may extract the at least one feature from the data based on information about the at least one feature to be used to train a model for determining whether to synchronize data, which is stored in a memory (e.g., the memory 440 of FIG. 4). According to an embodiment, the model and the information about the at least one feature may be stored in the memory of the electronic device. According to an embodiment, the electronic device may receive the model and the information about the at least one feature from a first external electronic device (e.g., the first external electronic device 402 of FIG. 4) through the communication circuit and may store the received model and at least one feature in the memory. According to another embodiment, the model and the information about the at least one feature may be stored in the electronic device while the electronic device is manufactured.

According to an embodiment, the model may be a model trained by using training data in which at least one feature is used as input data and a classification value about whether to synchronize is used as output data. According to an embodiment, the at least one feature may include an output value of at least one sensor designated for a particular kind of health information service.

According to an embodiment, the model may include an equation in which the at least one feature is used as an independent variable, the classification value about whether to synchronize is used as a dependent variable, and a quantified correlation (or correlation relationship) between independent variables. According to an embodiment, the model may be trained by using various supervised learning algorithms (e.g., an MLR algorithm, a CNN algorithm, or an RNN algorithm) depending on the complexity of variables. According to an embodiment, the model may be an MLR model trained by using the MLR algorithm. For example, the model may be "y = b₀ + b_{1·}x₁ + b_{2·}x₂ +...+ b_{n·}xₙ". Here, "y" may be the result; b₀ may be a bias; x₁, x₂... xₙ₋₁, and xₙ may be input variables; b₁, b₂... bₙ₋₁, and bₙ may be weights. According to an embodiment, the model stored in the electronic device may be a model in which the weights b₀ to bₙ are set to specified values. According to an embodiment, the input variables x₁ to xₙ may correspond to the at least one feature. According to an embodiment, the information about the at least one feature stored in the electronic device may include information indicating whether each of the input variables x₁ to xₙ is an output value of a particular sensor.

According to an embodiment, the electronic device may extract the at least one feature from the data obtained in operation 501, based on the information about the at least one feature. According to an embodiment, the electronic device may extract the at least one feature from data obtained by using at least one sensor of the electronic device or the second external electronic device. According to an embodiment, the electronic device may extract an output value of at least one sensor designated for a particular health information service from the data obtained from various sensors of the electronic device or the second external electronic device.

In operation 505, the electronic device may determine whether to synchronize the data, based on the model. According to an embodiment, the electronic device may determine whether to synchronize the data, based on the model stored in the memory by using the at least one feature extracted from the data. For example, the model may be "y = b₀ + b_{1·}x₁ + b_{2·}x₂ +...+ b_{n·}xₙ". According to an embodiment, the electronic device may calculate the result "y" by inputting the at least one feature extracted from the data to the input variables x₁ to xₙ corresponding to each feature. According to an embodiment, the electronic device may determine whether to synchronize the data, depending on whether the result "y" is greater than or equal to a specified value (e.g., y₀) or is smaller than the specified value. For example, when the result "y" is greater than or equal to the specified value, the electronic device may determine to synchronize the data; when the result "y" is smaller than the specified value, the electronic device may determine not to synchronize data.

In operation 507, the electronic device may send the data. According to an embodiment, when the data are determined to be synchronized, the electronic device may send the data through the communication circuit to a first external electronic device (e.g., the external electronic device 402 of FIG. 4) providing the health information service. According to an embodiment, when the data are determined to be synchronized, the electronic device may process the data and may send the processed data to the first external electronic device.

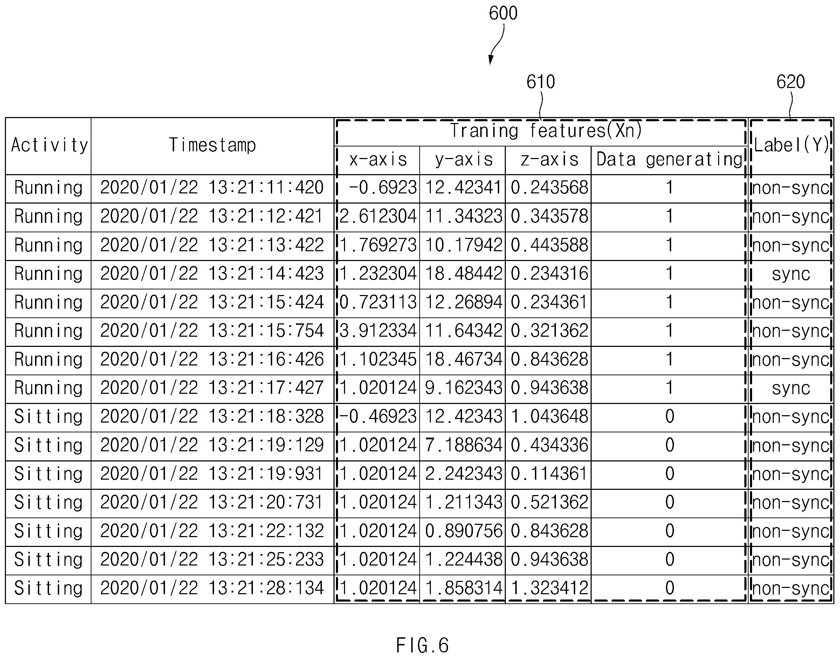

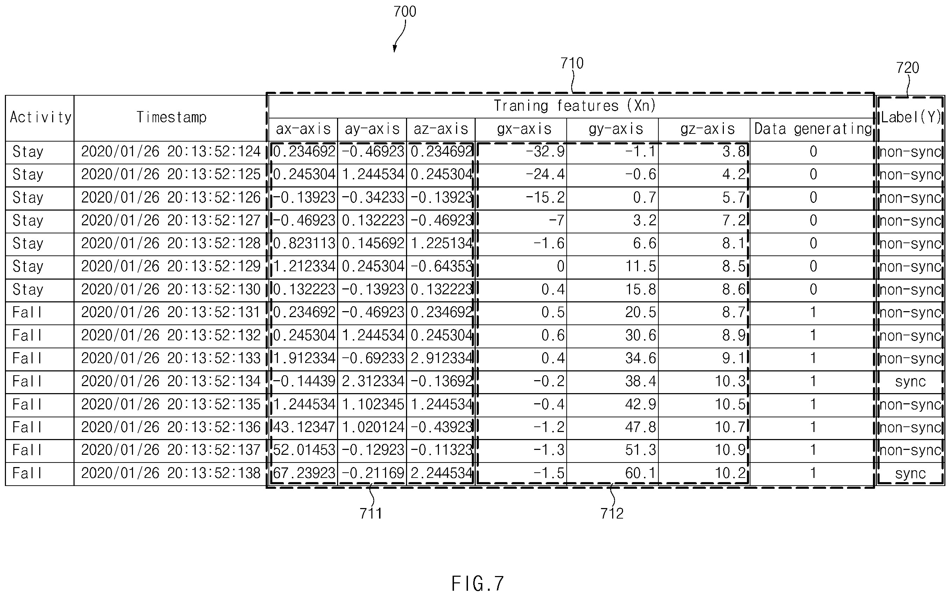

Below, training data that are used to create a model to be stored in an electronic device according to an embodiment will be described with reference to FIGs. 6 and 7.

FIG. 6 is a diagram 600 illustrating training data for creating a model to be stored in an electronic device according to an embodiment. Operations of an electronic device (e.g., the electronic device 401 of FIG. 4) to be described below may be performed by a processor (e.g., the processor 450 of FIG. 4) of the electronic device.

According to an embodiment, the model stored in the electronic device may be created by the electronic device or may be received from an external electronic device (e.g., the first external electronic device 402 of FIG. 4) after being created by the external electronic device.

According to an embodiment, the electronic device or the external electronic device may collect training data for creating a model. According to an embodiment, variables of the data may include the type of data necessary for the particular health information service, the generating condition (or generating time point) of the data, and/or the synchronization condition (or synchronization time point) of the data. According to an embodiment, depending on these variables, the electronic device or the external electronic device may collect data about the health information service that are generated by using at least one sensor. According to an embodiment, the at least one sensor may be a sensor that is mounted in the electronic device or an external electronic device (e.g., the second external electronic device 403 of FIG. 4) communicating with the electronic device.

According to an embodiment, training data illustrated in FIG. 6 may be data collected to create and train a model for determining whether to synchronize data associated with an intense workout monitoring service. According to an embodiment, the electronic device or the external electronic device may extract, as a feature, an output value of at least one sensor that is collected when a specified activity defined based on a criterion (or condition) for providing contents at the health information service is made. For example, the criterion for providing contents at the health information service may include at least one of a time or a place. For example, the condition for providing contents at the health information service may include a state of the user or the type of motion. For example, in the case where the health information service is an intense workout monitoring service, the condition for providing contents at the health information service may include at least one of "running," "walking," "sitting," or "sleep." According to an embodiment, the electronic device or the external electronic device may extract, as the feature, the output value of at least one sensor that is collected when an intense workout activity is detected. Referring to FIG. 6, for example, the electronic device or the external electronic device may extract, as the feature, three axis (i.e., x-axis, y-axis, and z-axis) values of an acceleration sensor that are collected when running motion of a specified speed or higher is made.

According to an embodiment, the electronic device or the external electronic device may determine whether to generate data (Data generating: 1 or 0) depending on a generating time point (or generating condition) of data designated in the intense workout monitoring service. Here, the data may refer to data to be sent to the server that provides the intense workout monitoring service. According to an embodiment, the electronic device or the external electronic device may use, as a feature, data about whether to generate data.

According to an embodiment, the electronic device or the external electronic device may extract a feature from pieces of data at a plurality of time points. According to an embodiment, the features extracted from the pieces of data of the plurality of time points may be referred to as a "feature set 610". According to an embodiment, the electronic device or the external electronic device may generate a training data set in which the feature set 610 is used as an input data set.

According to an embodiment, with regard to the feature set 610, the electronic device or the external electronic device may label a classification value (Label(Y): sync or non-sync) about whether to synchronize data, depending on a synchronization time point (or synchronization condition) of data designated in the intense workout monitoring service. According to an embodiment, the electronic device or the external electronic device may label the classification value about whether to synchronize data, for each data corresponding to the feature set 610. Here, the data corresponding to the feature set 610 may be classified depending on the time points at which data are obtained (or generated). According to an embodiment, classification values about whether to synchronize, which are labeled with respect to the feature set 610, may be referred to as a "label set 620".

According to an embodiment, the electronic device or the external electronic device may train a model by using training data in which the feature set 610 is used as an input data set and the label set 620 is used as an output data set. According to an embodiment, the model may be the MLR model in which xₙ is an independent variable (or input variable) and "Y" is a dependent variable (or output variable). For example, the electronic device or the external electronic device may generate a model being "Y = B₀ + B_{1·}X₁ + B_{2·}X₂ + B_{3·}X₃ + B_{4·}X₄" and information about at least one feature indicating that X₁ is the x-axis value of an acceleration sensor, X₂ is the y-axis value of the acceleration sensor, and X₃ is the z-axis value of the acceleration sensor, and X₄ indicates whether to generate data (Data generating: 1 or 0). For example, the electronic device or the external electronic device may obtain an equation of relationship between B₀, B₁, B₂, B₃, and B₄ by inputting a value corresponding to each feature of the input data set to Xₙ and inputting a value corresponding to the output data set to "Y" and may quantize B₀, B₁, B₂, B₃, and B₄ based on the obtained equation of relationship. According to an embodiment, the electronic device or the external electronic device may create a model by obtaining the equation in which B₀, B₁, B₂, B₃, and B₄ are quantized or determined, that is, "Y = B₀ + B_{1·}X₁ + B_{2·}X₂ + B_{3·}X₃ + B_{4·}X₄".

FIG. 7 is a diagram 700 illustrating training data for creating a model to be stored in an electronic device according to an embodiment. Operations of an electronic device (e.g., the electronic device 401 of FIG. 4) to be described below may be performed by a processor (e.g., the processor 450 of FIG. 4) of the electronic device.

According to an embodiment, a model stored in the electronic device may be created by the electronic device or may be received from an external electronic device (e.g., the first external electronic device 402 of FIG. 4) after being created by the external electronic device.

According to an embodiment, the electronic device or the external electronic device may collect training data for creating a model. According to an embodiment, training data illustrated in FIG. 7 may be data collected to create and train a model for determining whether to synchronize data associated with a fall monitoring service. According to an embodiment, the electronic device or the external electronic device may extract, as a feature, an output value of at least one sensor that is collected when a motion is determined to correspond to a certain probability of falling. Referring to FIG. 7, for example, the electronic device or the external electronic device may extract, as the feature, three axis (i.e., ax-axis, ay-axis, and az-axis) values of an acceleration sensor and three axis (i.e., gx-axis, gy-axis, and gz-axis) values of a gyro sensor, which are collected when the falling motion is made.

According to an embodiment, the electronic device or the external electronic device may determine whether to generate data (Data generating: 1 or 0) depending on a generating time point (or generating condition) of data designated in the fall monitoring service. Here, the data may refer to data to be sent to a server that provides the fall monitoring service. According to an embodiment, the electronic device or the external electronic device may use, as a feature, data about whether to generate data.

According to an embodiment, the electronic device or the external electronic device may extract a feature from pieces of data at a plurality of time points. According to an embodiment, the features extracted from the pieces of data of the plurality of time points may be referred to as a "feature set 710". According to an embodiment, the electronic device or the external electronic device may generate a training data set in which the feature set 710 is used as an input data set.

According to an embodiment, with regard to the feature set 710, the electronic device or the external electronic device may label a classification value (Label(Y): sync or non-sync) about whether to synchronize data, depending on a synchronization time point (or synchronization condition) of data designated in the falling monitoring service. According to an embodiment, the electronic device or the external electronic device may label the classification value about whether to synchronize data, for each data corresponding to the feature set 710. Here, the data corresponding to the feature set 710 may be classified depending on the time points at which data are obtained (or generated). According to an embodiment, classification values about whether to synchronize, which are labeled with respect to the feature set 710, may be referred to as a "label set 720".

According to an embodiment, the electronic device or the external electronic device may train a model by using training data in which the feature set 710 is used as an input data set and the label set 720 is used as an output data set. According to an embodiment, the model may be the MLR model in which Xₙ is an independent variable (or input variable) and "Y" is a dependent variable (or output variable). For example, the electronic device or the external electronic device may generate a model being "Y = B₀+ B_{1·}X₁ + B_{2·}X₂ + B_{3·}X₃ + B_{4·}X₄ + B_{5·}X₅ + B_{6·}X₆+ B_{7·}X₇," where X₁ is the x-axis value of the acceleration sensor, X₂ is the y-axis value of the acceleration sensor, X₃ is the z-axis value of the acceleration sensor, X₄ is the x-axis value of the gyro sensor, X₅ is the y-axis value of the gyro sensor, X₆ is the z-axis value of the gyro sensor, and X₇ indicates whether to generate data (Data generating: 1 or 0).

According to an embodiment, the at least one feature may include output values of a plurality of different sensors. Referring to FIG. 7, for example, the at least one feature may include the output value 711 of the acceleration sensor and the output value 712 of the gyro sensor. According to an embodiment, when at least one feature includes output values of a plurality of different sensors, the electronic device or the external electronic device may normalize the plurality of features before training the model. According to an embodiment, the output values of the plurality of different sensors may differ from each other in scale. According to an embodiment, when scales of the plurality of features are different, the electronic device or the external electronic device may perform scaling processing such that the scales of the plurality of features are normalized. According to an embodiment, when a plurality of features include output values of a plurality of different sensors, the electronic device or the external electronic device may generate the feature set 710 in which output values are formed in a plurality of dimensions for each sensor. According to an embodiment, when a plurality of features include output values of a plurality of different sensors, the electronic device or the external electronic device may perform dimensionality reduction processing on the plurality of features. According to an embodiment, the electronic device or the external electronic device may generate a normalized feature set (not illustrated) by performing at least one of scaling processing or dimensionality reduction processing on the feature set 710. According to an embodiment, the electronic device or the external electronic device may use the normalized feature set (not illustrated) as an input data set.

For example, the electronic device or the external electronic device may obtain an equation of relationship between B₀, B₁, B₂, B₃, B₄, B₅, B₆, and B₇ by inputting a value corresponding to each normalized feature of the input data set to Xₙ and inputting a value corresponding to the output data set to "Y" and may quantize B₀, B₁, B₂, B₃, B₄, B₅, B₆, and B₇ based on the obtained equation of relationship. According to an embodiment, the electronic device or the external electronic device may create a model by obtaining the equation in which B₀, B₁, B₂, B₃, B₄, B₅, B₆, and B₇ are quantized or determined, that is, "Y = B₀ + B_{1·}X₁ + B_{2·}X₂ + B_{3·}X₃ + B_{4·}X₄ + B_{5·}X₅ + B_{6·}X₆ + B_{7·}X₇".

According to an embodiment, when the electronic device determines whether to synchronize data newly obtained by using a sensor based on the created model, the electronic device may perform scaling or dimensionality reduction processing on at least one feature extracted from the data and may determine whether to synchronize the data based on the model by using the processed feature.

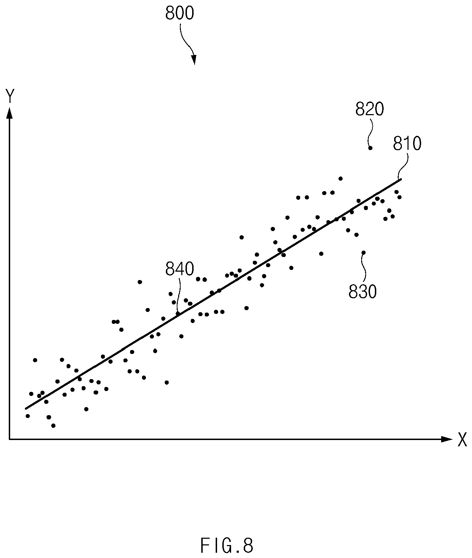

Below, how to determine whether to synchronize data based on a model according to an embodiment will be described with reference to FIG. 8.

FIG. 8 is a diagram 800 for describing an operation of an electronic device according to an embodiment. Operations of an electronic device to be described below may be performed by the electronic device 401 of FIG. 4 or the processor 450 of the electronic device 401.

According to an embodiment, a model that the electronic device uses may be a multiple linear regression (MLR) model. For example, the model may be "Y = B₀ + B_{1·}X₁ + B_{2·}X₂ +...+ B_{n·}Xₙ". Here, "Y" may be a result; B₀ may be a bias; X₁, x₂... Xₙ₋₁, and Xₙ may be input variables; B₁, B₂...Bₙ₋₁, and Bₙ may be weights. According to an embodiment, in the model, the weights B₁, B₂...Bₙ₋₁, and Bₙ may be determined as numerical values.

According to an embodiment, the input variables X₁ to Xₙ of the model may correspond to the X-axis of a graph, and the result "Y" of the model may correspond to the Y-axis of the graph. According to an embodiment, the electronic device may calculate the result "Y" by inputting the at least one feature extracted from the data to the input variables X₁ to Xₙ corresponding to each feature. According to an embodiment, the electronic device may determine (or classify) whether to synchronize the data, depending on whether the result "Y" is greater than or equal to the specified value or is smaller than the specified value. For example, the specified value may be "Y₀". According to an embodiment, a straight line 810 may be a set of points each being "Y = B₀ + B_{1·}X₁ + B_{2·}X₂ +...+ B_{n·}Xₙ = Y₀". According to an embodiment, in the graph, points placed above the straight line 810 may be points each "Y = B₀ + B_{1·}X₁ + B_{2·}X₂ +...+ B_{n·}Xₙ > Y₀". According to an embodiment, in the graph, points placed below the straight line 810 may be points each "Y = B₀ + B_{1·}X₁ + B_{2·}X₂ +...+ B_{n·}Xₙ < Y₀". According to an embodiment, the electronic device may determine a point 820 marked on the straight line 810 and above the straight line 810 as a point to synchronize data and may determine a point 830 marked below the straight line 810 so a point to not synchronize data. According to an embodiment, in the case of data corresponding to a point 840 that is marked close to the straight line 810 within the specified threshold value, the electronic device may determine that the reliability is smaller than the specified threshold value. According to an embodiment, when the reliability is smaller than the specified threshold value, the electronic device may display a user interface requesting feedback of the user associated with whether to synchronize data on a display and may determine (or classify) whether to synchronize based on an input received through the user interface.

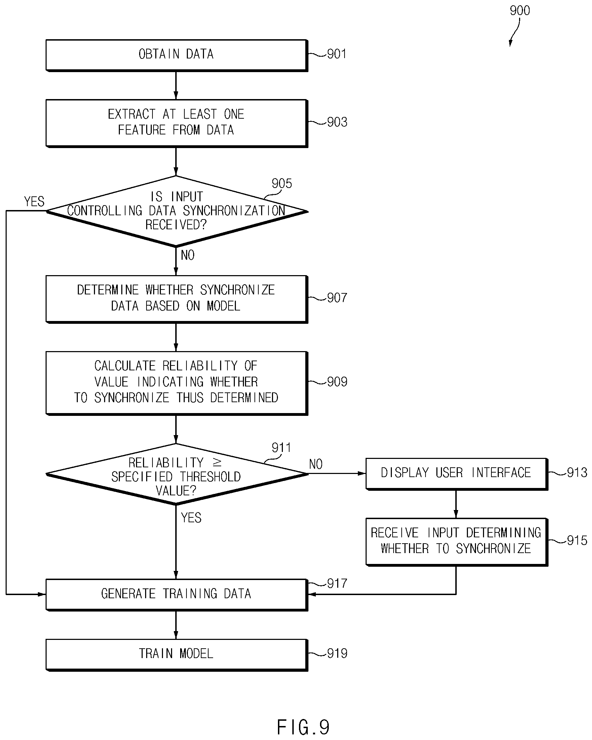

Below, an operation in which an electronic device according to an embodiment trains a model will be described with reference to FIG. 9.

FIG. 9 is a flowchart 900 illustrating an operation of an electronic device according to an embodiment. Operations of an electronic device to be described below may be performed by the electronic device 401 of FIG. 4 or the processor 450 of the electronic device 401.

In operation 901, the electronic device may obtain data. According to an embodiment, operation 901 may correspond to operation 501 of FIG. 5. The description given with reference to operation 501 may be identically applied to description associated with operation 901.

In operation 903, the electronic device may extract at least one feature from the data. According to an embodiment, operation 903 may correspond to operation 503 of FIG. 5. The description given with reference to operation 503 may be identically applied to description associated with operation 903.

In operation 905, the electronic device may determine whether an input allowing data to be synchronized is received. According to another embodiment, the electronic device may receive an input allowing data to be synchronized from the user, regardless of the determination to synchronize data based on the model. For example, the electronic device may receive an input indicating (or directing) synchronization of data of a time point designated by the user (i.e., data obtained by using a sensor at the designated time point). According to an embodiment, when it is determined that the input allowing data to be synchronized is received (operation 905-YES), the electronic device may perform operation 917; when it is determined that the input allowing data to be synchronized is not received (operation 905-NO), the electronic device may perform operation 907. According to another embodiment, the electronic device may perform operation 903 after performing operation 905.

In operation 907, the electronic device may determine whether to synchronize, based on the model. According to an embodiment, operation 907 may correspond to operation 505 of FIG. 5. The description given with reference to operation 505 may be identically applied to description associated with operation 907. According to an embodiment, the model may be the MLR model. For example, the model may be "y = b₀ + b_{1·}x₁ + b_{2·}x₂ +...+ b_{n·}xₙ". According to an embodiment, the electronic device may input the extracted at least one feature to the input variables x₁ to xₙ corresponding to each feature. According to an embodiment, the electronic device may determine whether to synchronize data, depending on whether the result "y" calculated by inputting the at least one feature extracted from the data to the input variables x₁ to xₙ is greater than or equal to the specified value (e.g., y₀) or is smaller than the specified value. For example, when the result "y" is greater than or equal to the specified value, the electronic device may determine to synchronize the data; when the result "y" is smaller than the specified value, the electronic device may determine not to synchronize data.

In operation 909, the electronic device may calculate a reliability of a value indicating whether to synchronize thus determined. According to an embodiment, the electronic device may determine the reliability of the result of predicting whether to synchronize, based on the difference between the result "y" calculated by inputting the at least one feature extracted from the data to the input variables x₁ to xₙ and the specified value for determining whether to synchronize. According to an embodiment, as the difference between the calculated result "y" and the specified value becomes greater, the reliability may increase.

In operation 911, the electronic device may determine whether the reliability is greater than or equal to the specified threshold value. According to an embodiment, the specified value may be assumed to be y₀. According to an embodiment, the electronic device may determine whether the difference |y-y₀| between the calculated result "y" and the specified value is greater than or equal to the specified threshold value "a". According to an embodiment, when the difference |y-y₀| between the calculated result "y" and the specified value is greater than or equal to the specified threshold value "a" (operation 911-YES: |y-y₀|≥a), the electronic device may perform 917; when the difference |y-y₀| between the calculated result "y" and the specified value is smaller than the specified threshold value "a" (operation 911-NO: |y-y₀|<a), the electronic device may perform operation 913. According to an embodiment, when "NO" is determined in operation 911, the electronic device may postpone data synchronization and may perform operation 913.

In operation 913, the electronic device may display a user interface. According to an embodiment, the electronic device may display a user interface requesting feedback of the user associated with whether to synchronize data, on a display (e.g., the display 430 of FIG. 4).

In operation 915, the electronic device may receive an input indicating (or directing) whether to synchronize. According to an embodiment, the electronic device may receive the input indicating whether to synchronize data, from the user through the user interface. According to an embodiment, when the electronic device receives the input indicating (or directing) data synchronization through the user interface, the electronic device may synchronize data. According to an embodiment, when the electronic device receives an input indicating no data synchronization through the user interface, even though data synchronization is determined as being performed based on the model, the electronic device may not synchronize data.

In operation 917, the electronic device may generate training data. According to an embodiment, the electronic device may generate training data in which at least one feature extracted from data is used as input data and a value indicating whether to synchronize thus determined is used as output data. According to an embodiment, in the case where the electronic device performs operation 905 and then performs operation 917 without performing operation 907 to operation 915, the electronic device may generate training data in which the at least one feature extracted from the data is used as input data and a value indicating that the data are targeted for synchronization is used as output data. According to an embodiment, in the case where the electronic device performs operation 911 and then performs operation 917 without performing operation 913 and operation 915, the electronic device may generate training data in which the at least one feature extracted from the data is used as input data and a value indicating whether to synchronize determined based on the model is used as output data. According to an embodiment, in the case where the electronic device performs operation 911, performs operation 913 and operation 915, and then performs operation 917, the electronic device may generate training data in which the at least one feature extracted from the data is used as input data and a value indicating whether to synchronize determined based on the input received through the user interface is used as output data.

In operation 919, the electronic device may train the model. According to an embodiment, the electronic device may train the model by using the generated training data. According to an embodiment, the electronic device may additionally train the initial model stored in a memory (e.g., the memory 440 of FIG. 4) by using the generated training data. For example, the initial model stored in the memory 440 may be "y = b₀ + b_{1·}x₁ + b_{2·}x₂ +... + b_{n·}xₙ", a bias of the initial model may be b₀, and weights of the initial model may be b₁ to bₙ. According to an embodiment, the electronic device may obtain the new bias b₀' and the new weights b₁' to bₙ' by additionally training the model. According to an embodiment, the electronic device may obtain an updated model "y = b₀' + b₁'·x₁ + b₂'·x₂ +...+ bₙ'·xₙ" by additionally training the model by using training data.

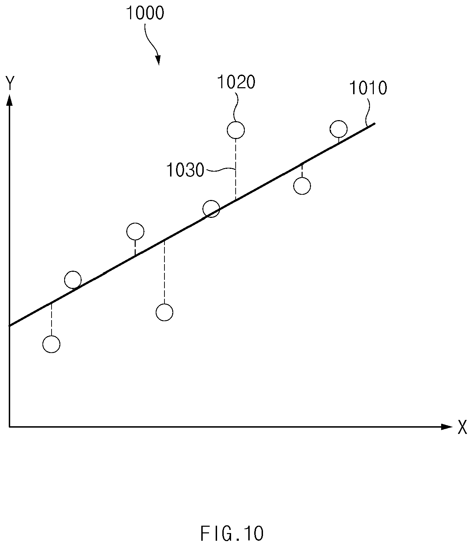

Below, an operation in which an electronic device according to an embodiment calculates a reliability of a value indicating whether to synchronize determined based on a mode will be described with reference to FIG. 10.

FIG. 10 is a diagram 1000 for describing an operation of an electronic device according to an embodiment. Operations of an electronic device to be described below may be performed by the electronic device 401 of FIG. 4 or the processor 450 of the electronic device 401.

According to an embodiment, the model that the electronic device uses may be the multiple linear regression (MLR) model. For example, the model may be "Y = B₀ + B_{1·}X₁ + B_{2·}X₂ +...+ B_{n·}Xₙ. Here, "Y" may be a result; B₀ may be a bias; x₁, x₂... Xₙ₋₁, and Xₙ may be input variables; B₁, B₂ ...Bₙ₋₁, and Bₙ may be weights. According to an embodiment, in the model, the weights B₁, B₂ ...Bₙ₋₁, and Bₙ may be determined as numerical values.

According to an embodiment, the input variables X₁ to Xₙ of the model may correspond to the X-axis of the graph, and the result "Y" of the model may correspond to the Y-axis of the graph. According to an embodiment, the electronic device may calculate the result "y" by inputting at least one feature extracted from data to the input variables x₁ to xₙ corresponding to each feature. According to an embodiment, the electronic device may determine (or classify) whether to synchronize the data, depending on whether the result "Y" is greater than or equal to the specified value or is smaller than the specified value. For example, the specified value may be Y₀. According to an embodiment, the straight line 1010 may be a set of points each being "Y = B₀ + B_{1·}X₁ + B_{2·}X₂ +... + B_{n·}Xₙ = Y₀". According to an embodiment, in the graph, points placed above the straight line 1010 may be points each "Y = B₀ + B_{1·}X₁ + B_{2·}X₂ +... + B_{n·}Xₙ > Y₀". According to an embodiment, in the graph, points placed below the straight line 1010 may be points each "Y = B₀ + B_{1·}X₁ + B_{2·}X₂ +...+ B_{n·}Xₙ < Y₀". According to an embodiment, the electronic device may determine a point marked on the straight line 1010 and above the straight line 1010 so a point to synchronize data and may determine a point marked below the straight line 1010 as a point to not synchronize data.

According to an embodiment, a plurality of points marked in the graph may indicate the value "Y" indicating whether to synchronize data, which the electronic device determines based on the model. For example, the result value "Y" about whether to synchronize determined based on the model, which is indicated by a first point 1020, may be Y₁. According to an embodiment, the electronic device may calculate a reliability of the first point 1020 by calculating the difference |Y₁-Y₀| between the result value Y₁ and the specified value Y₀. According to an embodiment, in the graph, because the straight line 1010 is a set of points at which the result value Y about whether to synchronize determined based on the model is Y₀, the reliability of the first point 1020 may mean the Y-axis distance 1030 from the first point 1020 to the straight line 1010. According to an embodiment, the electronic device may calculate the reliability of each point based on the Y-axis distance from each point marked in the graph to the straight line 1010. According to an embodiment, as the Y-axis distance from a point to the straight line 1010 increases, the reliability may become higher. According to an embodiment, the reliability of points marked distant to the straight line 1010 on the graph may be great (or high), and the reliability of points marked close from the straight line 1010 on the graph may be small (or low). According to an embodiment, the electronic device may display a user interface requesting feedback of the user associated with whether to synchronize data corresponding to the point at which the Y-axis distance from the straight line 1010 is smaller than a specified threshold value, on a display (e.g., the display 430 of FIG. 4).

According to embodiments of the disclosure, an electronic device (e.g., the electronic device 101 of FIG. 1 or the electronic device 401 of FIG. 4) may include a communication circuit (e.g., the communication circuit 190 of FIG. 1 or the communication circuit 410 of FIG. 4), a sensor (e.g., the sensor module 176 of FIG. 1 or the sensor 420 of FIG. 4), a display (e.g., the display module 160 of FIG. 1 or the display 430 of FIG. 4), a memory (e.g., the memory 130 of FIG. 1 or the memory 440 of FIG. 4), and a processor (e.g., the processor 120 of FIG. 1 or the processor 450 of FIG. 4) that is operatively connected with the communication circuit, the sensor, the display, and the memory. The memory may store one or more instructions that, when executed, cause the processor to obtain data associated with a health information service by using the sensor, to extract, from the data, at least one feature to be used to train a model for determining whether to synchronize the data, based on information stored in the memory and associated with the at least one feature, to determine whether to synchronize the data based on the model stored in the memory by using the at least one feature, and to send the data to a first external electronic device (e.g., the electronic device 102 of FIG. 1, the electronic device 104 of FIG. 1, the server 108 of FIG. 1, or the first external electronic device 402 of FIG. 4) providing the health information service through the communication circuit, in response to a determination to synchronize the data.

According to an embodiment of the disclosure, the instructions, when executed, may cause the processor to receive the model and the information about the at least one feature from the first external electronic device through the communication circuit, and to store the model and the information about the at least one feature thus received in the memory.

According to an embodiment of the disclosure, the model may be trained by using training data in which the at least one feature is used as input data and a classification value about whether to synchronize is used as output data, and the at least one feature may include an output value of the sensor designated for the health information service. The electronic device may include a plurality of sensors each designated for a particular kind of health information service.

According to an embodiment of the disclosure, at least one of scaling processing or dimensionality reduction processing may be performed on the at least one feature.

According to an embodiment of the disclosure, the instructions, when executed, may cause the processor to process the data to be sent to the first external electronic device, and to send the processed data to the first external electronic device.

According to an embodiment of the disclosure, the instructions, when executed, may cause the processor to receive data obtained by using a sensor of a second external electronic device (e.g., the electronic device 102 of FIG. 1, the electronic device 104 of FIG. 1, the server 108 of FIG. 1, or the second external electronic device 403 of FIG. 4) through the communication circuit from the second external electronic device, and to determine whether to synchronize the received data based on the model.

According to an embodiment of the disclosure, the instructions, when executed, may cause the processor to generate training data in which the at least one feature extracted from the data for which whether to synchronize is determined based on the model is used as input data and a value indicating the determination of whether to synchronize the data is used as output data, and to train the model by using the training data.

According to an embodiment of the disclosure, the instructions, when executed, may cause the processor to calculate a reliability of a determination of whether to synchronize the data based on the model, to display a user interface requesting a feedback of a user associated with whether to synchronize the data on the display, when the reliability is smaller than a specified threshold value, to receive an input indicating synchronization of the data from the user through the user interface, and to send the data to the first external electronic device in response to the input indicating synchronization of the data being received.

According to an embodiment of the disclosure, the instructions, when executed, may cause the processor to generate training data in which the at least one feature extracted from the data for which whether to synchronize is determined based on the input is used as input data and a value indicating the determination of whether to synchronize, which corresponds to the input, is used as output data, and to train the model by using the training data.

According to an embodiment of the disclosure, the instructions, when executed, may cause the processor to extract the at least one feature from the obtained data based on the information about the at least one feature, in response to receiving from a user an input allowing the obtained data to be synchronized, to generate training data in which the at least one feature is used as input data and a value indicating that the obtained data is to be synchronized is used as output data, and to train the model by using the training data.

According to an embodiment of the disclosure, an operating method of an electronic device (e.g., the electronic device 101 of FIG. 1 or the electronic device 401 of FIG. 4) may include obtaining data associated with a health information service by using a sensor (e.g., the sensor module 176 of FIG. 1 or the sensor 420 of FIG. 4), extracting at least one feature from the data based on information about the at least one feature to be used to train a model for determining whether to synchronize the data, determining whether to synchronize the data based on the model by using the at least one feature, and sending the data to a first external electronic device (e.g., the electronic device 102 of FIG. 1, the electronic device 104 of FIG. 1, the server 108 of FIG. 1, or the first external electronic device 402 of FIG. 4) providing the health information service, in response to a determination to synchronize the data.

According to an embodiment of the disclosure, the method may further include receiving the model and the information about the at least one feature from the first external electronic device, and storing the model and the information about the at least one feature thus received in a memory of the electronic device.

According to an embodiment of the disclosure, the model may be trained by using training data in which the at least one feature is used as input data and a classification value about whether to synchronize is used as output data, and the at least one feature may include an output value of the sensor designated for the health information service. The electronic device may include a plurality of sensors each designated for a particular kind of health information service.

According to an embodiment of the disclosure, at least one of scaling processing or dimensionality reduction processing may be performed on the at least one feature.

According to an embodiment of the disclosure, the method may further include processing the data to be sent to the first external electronic device, and sending the processed data to the first external electronic device.

According to an embodiment of the disclosure, the method may further include receiving data obtained by using a sensor of a second external electronic device (e.g., the electronic device 102 of FIG. 1, the electronic device 104 of FIG. 1, the server 108 of FIG. 1, or the second external electronic device 403 of FIG. 4) from the second external electronic device, and determining whether to synchronize the received data based on the model.

According to an embodiment of the disclosure, the method may further include generating training data in which the at least one feature extracted from the data for which whether to synchronize is determined based on the model is used as input data and a value indicating the determination of whether to synchronize the data is used as output data, and training the model by using the training data.

According to an embodiment of the disclosure, the method may further include calculating a reliability of a determination of whether to synchronize the data based on the model, displaying a user interface requesting a feedback of a user associated with whether to synchronize the data on a display (e.g., the display module 160 of FIG. 1 or the display 430 of FIG. 4) of the electronic device, when the reliability is smaller than a specified threshold value, receiving an input indicating synchronization of the data from the user through the user interface, and sending the data to the first external electronic device in response to the input indicating synchronization of the data being received.

According to an embodiment of the disclosure, the method may further include generating training data in which the at least one feature extracted from the data for which whether to synchronize is determined based on the input is used as input data and a value indicating the determination of whether to synchronize, which corresponds to the input, is used as output data, and training the model by using the training data.

According to an embodiment of the disclosure, the method may further include extracting the at least one feature from the obtained data based on the information about the at least one feature, in response to receiving from a user an input allowing the obtained data to be synchronized, generating training data in which the at least one feature is used as input data and a value indicating that the obtained data is to be synchronized is used as output data, and training the model by using the training data.

Certain of the above-described embodiments of the present disclosure can be implemented in hardware, firmware or via the execution of software or computer code that can be stored in a recording medium such as a CD ROM, a Digital Versatile Disc (DVD), a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein.

While the present disclosure has been shown and described with reference to various embodiments thereof, it will be understood by those skilled in the art that various changes in form and details may be made therein without departing from the present disclosure as defined by the appended claims and their equivalents.

## Claims

1. An electronic device comprising:
a communication circuit;
a sensor;
a display;
a memory; and
a processor operatively connected with the communication circuit, the sensor, the display, and the memory,
wherein the memory stores one or more instructions that, when executed, cause the processor to:
obtain data associated with a health information service by using the sensor;
based on information stored in the memory and about at least one feature to be used to train a model for determining whether to synchronize the data, extract the at least one feature from the data;
determine whether to synchronize the data based on the model stored in the memory by using the at least one feature; and
in response to a determination to synchronize the data, send the data to a first external electronic device providing the health information service through the communication circuit.

2. The electronic device of claim 1, wherein the instructions, when executed, cause the processor to:
receive the model and the information about the at least one feature from the first external electronic device through the communication circuit; and
store the model and the information about the at least one feature thus received in the memory.

3. The electronic device of claim 1, wherein the model is trained by using training data in which the at least one feature is used as input data and a classification value about whether to synchronize is used as output data,
wherein the at least one feature includes an output value of the sensor designated for the health information service, and
wherein the electronic device includes a plurality of sensors each designated for a particular kind of health information service.

4. The electronic device of claim 3, wherein scaling processing and/or dimensionality reduction processing is performed on the at least one feature.

5. The electronic device of claim 1, wherein the instructions, when executed, cause the processor to:
process the data to be sent to the first external electronic device; and
send the processed data to the first external electronic device.

6. The electronic device of claim 1, wherein the instructions, when executed, cause the processor to:
receive data obtained by using a sensor of a second external electronic device through the communication circuit from the second external electronic device; and
determine whether to synchronize the received data based on the model.

7. The electronic device of claim 1, wherein the instructions, when executed, cause the processor to:
generate training data in which the at least one feature extracted from the data for which whether to synchronize is determined based on the model is used as input data and a value indicating a determination of whether to synchronize the data is used as output data; and
train the model by using the training data.

8. The electronic device of claim 1, wherein the instructions, when executed, cause the processor to:
calculate a reliability of a determination of whether to synchronize the data based on the model;
display a user interface requesting a feedback of a user associated with whether to synchronize the data on the display, when the reliability is smaller than a specified threshold value;
receive an input indicating synchronization of the data from the user through the user interface; and
send the data to the first external electronic device in response to the input indicating synchronization of the data being received.

9. The electronic device of claim 8, wherein the instructions, when executed, cause the processor to:
generate training data in which the at least one feature extracted from the data for which whether to synchronize is determined based on the input is used as input data and a value indicating the determination of whether to synchronize, which corresponds to the input, is used as output data; and
train the model by using the training data.

10. The electronic device of claim 1, wherein the instructions, when executed, cause the processor to:
extract the at least one feature from the obtained data based on the information about the at least one feature, in response to receiving from a user an input allowing the obtained data to be synchronized;
generate training data in which the at least one feature is used as input data and a value indicating that the obtained data is to be synchronized is used as output data; and
train the model by using the training data.

11. An operating method of an electronic device, the method comprising:
obtaining data associated with a health information service by using a sensor;
extracting at least one feature from the data based on information about the at least one feature to be used to train a model for determining whether to synchronize the data;
determining whether to synchronize the data based on the model by using the at least one feature; and
in response to a determination to synchronize the data, sending the data to a first external electronic device providing the health information service.

12. The method of claim 11, wherein the model is trained by using training data in which the at least one feature is used as input data and a classification value about whether to synchronize is used as output data,
wherein the at least one feature includes an output value of the sensor designated for the health information service, and
wherein the electronic device includes a plurality of sensors each designated for a particular kind of health information service.

13. The method of claim 11, further comprising:
generating training data in which the at least one feature extracted from the data for which whether to synchronize is determined based on the model is used as input data and a value indicating a determination of whether to synchronize the data is used as output data; and
training the model by using the training data.

14. The method of claim 11, further comprising:
calculating a reliability of a determination of whether to synchronize the data based on the model;
displaying a user interface requesting a feedback of a user associated with whether to synchronize the data on a display, when the reliability is smaller than a specified threshold value;
receiving an input indicating synchronization of the data from the user through the user interface; and
sending the data to the first external electronic device in response to the input indicating synchronization of the data being received.

15. The method of claim 11, further comprising:
extracting the at least one feature from the obtained data based on the information about the at least one feature, in response to receiving from a user an input allowing the obtained data to be synchronized;
generating training data in which the at least one feature is used as input data and a value indicating that the obtained data to be synchronized is used as output data; and
training the model by using the training data.
